# EUROPEAN PATENT SPECIFICATION

(11) **EP 2 639 240 B1**
(45) Date of publication and mention of the grant of the patent: **22.06.2016**
(21) Application number: 13155985.8
(22) Date of filing: 20.02.2013
(51) Int. Cl.: C07K 14/47

(54) **Peptides able to impair the inhibiting activity of MDM2/MDM4 heterodimer towards p53 and use thereof for cancer treatment**
Peptide zur Beeinträchtigung der Hemmung der Aktivität von MDM2/MDM4-Heterodimer zu p53 und Verwendung davon zur Krebsbehandlung
Peptides capables de neutraliser l'activité d'inhibition d'une hétérodimère MDM2/MDM4 vers p53 et leur utilisation pour le traitement du cancer

(30) Priority: 21.02.2012 IT RM20120060
(43) Date of publication of application: 18.09.2013
(73) Proprietor: Consiglio Nazionale Delle Ricerche, 00185 Roma (IT); Universita Degli Studi Di Perugia, 06123 Perugia (IT)
(72) Inventor: Pellegrino, Marsha, 00143 Roma (IT); Moretti, Fabiola, 00143 Roma (IT); Mancini, Francesca, 00143 Roma (IT); Macchiarulo, Antonio, 06123 Perugia (IT); Pellicciari, Roberto, 06123 Perugia (IT)
(74) Representative: Gitto, Serena

(56) References cited:
- ZOE VASILEIOU ET AL: "Convergent solid-phase and solution approaches in the synthesis of the cysteine-rich Mdm2 RING finger domain", JOURNAL OF PEPTIDE SCIENCE, vol. 15, no. 12, 1 December 2009 (2009-12-01), pages 824-831, XP055040405, ISSN: 1075-2617, DOI: 10.1002/psc.1182
- M. WADE ET AL: "Targeting Mdm2 and Mdmx in Cancer Therapy: Better Living through Medicinal Chemistry?", MOLECULAR CANCER RESEARCH, vol. 7, no. 1, 1 January 2009 (2009-01-01) , pages 1-11, XP055040406, ISSN: 1541-7786, DOI: 10.1158/1541-7786.MCR-08-0423
- WANG XINJIANG: "p53 regulation: teamwork between RING domains of Mdm2 and MdmX", CELL CYCLE, LANDES BIOSCIENCE, US, vol. 10, no. 24, 15 December 2011 (2011-12-15), pages 4225-4229, XP009163428, ISSN: 1551-4005
- K LINKE ET AL: "Structure of the MDM2/MDMX RING domain heterodimer reveals dimerization is required for their ubiquitylation in trans", CELL DEATH AND DIFFERENTIATION, vol. 15, no. 5, 1 May 2008 (2008-05-01), pages 841-848, XP055040408, ISSN: 1350-9047, DOI: 10.1038/sj.cdd.4402309
- R. K. SINGH ET AL: "Hetero-oligomerization with MdmX Rescues the Ubiquitin/Nedd8 Ligase Activity of RING Finger Mutants of Mdm2", JOURNAL OF BIOLOGICAL CHEMISTRY, vol. 282, no. 15, 1 April 2007 (2007-04-01), pages 10901-10907, XP055040409, ISSN: 0021-9258, DOI: 10.1074/jbc.M610879200
- ANTONIO MACCHIARULO ET AL: "Expanding the horizon of chemotherapeutic targets: From MDM2 to MDMX (MDM4)", MEDCHEMCOMM, vol. 2, no. 6, 1 January 2011 (2011-01-01) , page 455, XP055040410, ISSN: 2040-2503, DOI: 10.1039/c0md00238k
- DATABASE UniProt [Online] 19 October 2011 (2011-10-19), "SubName: Full=RNA polymerase II second largest subunit; Flags: Fragment;", XP002699738, retrieved from EBI accession no. UNIPROT:G0TA93 Database accession no. G0TA93

## Description

The present invention concerns peptides able to impair the inhibiting activity of MDM2/MDM4 heterodimer towards p53 and their use for cancer treatment. In particular, the invention concerns peptides able to inhibit MDM2/MDM4 heterodimer and maintain the association between MDM4 and p53 so to restore the p53 oncosuppressive function in cancer cells harboring wild type p53 protein, directing its function specifically towards an apoptotic outcome.

Inactivation of the oncosuppressor p53 by means of direct mutation of its gene is one of first genetic lesions recognized in human tumors (about 50% of human tumors). In tumors carrying wilde type p53, studies have evidenced alternative methods for p53 inactivation by deregulation of its partners, MDM4 and MDM2. The latter ones are two crucial regulators of the oncosuppressor p53. These two proteins carry out mainly inhibiting activity towards p53 by controlling both protein levels and transcriptional function.

The importance of MDM proteins on the inhibition of p53 activity resulted in development of different strategies aiming to disrupt the p53/MDM association.

For example WO 2008106507, WO201105219 patent applications are known and concern peptides inhibiting the interaction of p53 with MDM2 and MDM4, i.e. peptides aiming to break p53 binding with MDM4 and MDM2. According to WO201105219 patent application, the dissociation of p53 from MDM2 and MDM4 results in a biological response consisting both of proliferative arrest and cell death, although it is not specified whether the observed cell death is due to apoptosis.

WO2008106507 describes peptides able to bind with high affinity MDM2 and therefore, probably, inhibit the binding of MDM2 with all the members of the p53 family; however, such statement has not been experimentally proved. It is predicted, by computational analysis, that such peptides may bind MDM4 and therefore dissociate the same from p53. In vitro or in vivo experimental data have not been provided for a possible biological activity of these peptides. Therefore it is not predictable their effectiveness.

In addition, the strategy aiming to disrupt the binding of p53 to MDM4 and MDM2 does not consider recent studies suggesting that MDM4 binding to p53 potentiates its apoptotic activity. According to WO201105219 patent application, peptides would be able to inhibit the binding of p53 to MDM2 10 fold higher than to MDM4 (IC50=0.01 µM and 0.1 µM for MDM2 and MDM4, respectively). Therefore, it cannot be excluded that a part of the observed cell death, according to WO201105219, is due to the partial retention of MDM4-p53 complexes and not completely to the dissociation of p53 from MDM2 and MDM4.

WO200061193 patent application describes antisense compounds inhibiting MDM4 expression. Also in this case the importance of MDM4-p53 complex has not been taken into consideration for apoptosis induction.

Most promising results for reactivation of p53 are obtained using small molecules identified by high-throughput screening of compound libraries and drug design by computer analysis based on protein structure (Shangary S, Wang S. Targeting the MDM2-p53 interaction for cancer therapy. Clin Cancer Res. 2008 Sep 1; 14(17): 5318-24).

However, known therapeutic strategies show various disadvantages. For example, compounds dissociating MDM2 from p53 are ineffective in displacing MDM4 from p53 because of the intrinsic differences in p53 binding domains of MDM4 and MDM2. This could enhance apoptosis induction by MDM4-p53 complex. However, since such compounds do not inhibit the binding of MDM4 with MDM2, it can be predicted that the heterodimer still inhibits p53 activity. Moreover, association of MDM2 to MDM4 antagonizes the pro-apoptotic activity of MDM4 preventing this response. In addition growth arrest rather than apoptotic response often occurs, while thepreferred result in any cancer therapy is apoptosis (Toledo F, Wahl GM. MDM2 and MDM4: p53 regulators as targets in anticancer therapy. Int J Biochem Cell Biol. 2007; 39 (7-8): 1476-82. Epub 2007 Apr 8; Shangary S, Wang S. Targeting the MDM2-p53 interaction for cancer therapy. Clin Cancer Res. 2008 Sep 1; 14(17): 5318-24). Known methods, aiming to inhibit the interaction of p53 with MDM2 and/or MDM4, cause therefore activation of p53 not only towards apoptosis, as it would be preferred, but also towards proliferative arrest. In addition, the release of MDM2 from association with p53 results in higher degradative activity of MDM2 towards MDM4 and other proapoptotic molecules (as demonstrated by several recent papers).

All current methods failed to pass phase 1 of clinical trials, pointing out to existing limitations (Cheok CF, Verma CS, Baselga J, Lane DP. Translating p53 into the clinic. Nat Rev Clin Oncol. 2011 Jan;8(1):25-37. Epub 2010 Oct 26).

Based on these results, it emerges the need to provide new therapeutic methods for cancer treatment that overcomes the disadvantages of known methods.

Recent studies suggest that the MDM4/MDM2 heterodimer is the functional inhibitor of p53. This data suggest that agents aiming to inhibit MDM4/MDM2 heterodimer could represent new potential therapeutic agents. On the other hand, recent data suggest different activities of MDM2 and MDM4 under stress conditions. Under these conditions a weakening of association and degradation activity of MDM2 towards p53 mainly occurs (Marchenko ET to, 2007). In addition, upon a sub-lethal type of DNA damage, usually associated with growth arrest, MDM2 level increase and this results in degradation of MDM4 and some pro-apoptotic factors as HIPK2 (Rinaldo C, Prodosmo A, Mancini F, lacovelli S, Sacchi A, Moretti F, Soddu S. MDM2-regulated degradation of HIPK2 prevents p53Ser46 phosphorylation and DNA damage-induced apoptosis. Mol Cell. 2007 Mar 9;25(5):739-50). On the contrary, following a severe DNA damage, usually associated to apoptosis, levels of MDM2 do not increase or even decrease (Ashcroft M, Taya Y, Vousden KH Stress signals utilize multiple pathways to stabilize p53. Mol Cell Biol 200020: 3224-33; Latonen L, Taya Y, Laiho M UV-radiation induces dose-dependent regulation of p53 response and modulates p53-HDM2 interaction in human fibroblasts. Oncogene 2001 20: 6784-93; Meng LH, Kohn KW, Pommier Y Dose-response transition from cell cycle arrest to apoptosis with selective degradation of Mdm2 and p21WAF1/CIP1 in response to the novel anticancer agent, aminoflavone (NSC 686,288). Oncogene. 2007, 26, 4806-16).

Based on these data, a recent model considers the up-regulation of MDM2 occurring during cell growth arrest, an event favoring this response in comparison to cell death (Shmueli A, Oren M. Mdm2: p53's lifesaver? Mol Cell. 2007 Mar 23;25(6):794-6). On the other hand, MDM4 stabilizes p53, preventing the MDM2-mediated degradation, and promotes p53 mediated apoptosis under stress conditions (Jackson MW, Berberich SJ. MdmX protects p53 from Mdm2-mediated degradation. Mol Cell Biol 2000; 20:1001-7; Stad R, Little NA, Xirodimas DP, Frenk R, van der Eb AJ, Lane DP, et al. Mdmx stabilizes p53 and Mdm2 via two distinct mechanisms. EMBO Rep 2001; 2:1029-34; Mancini F, Gentiletti F, D'Angelo M, Giglio S, Nanni S, D'Angelo C, et al. MDM4 (MDMX) over-expression enhances stabilization of stress-induced p53 and promotes apoptosis. J Biol Chem 2004, 279:8169-80; Barboza JA, Iwakuma T, Terzian T, El-Naggar AK, Lozano G. Mdm2 and Mdm4 loss regulates distinct p53 activities. Mol Cancer Res 2008; 6:947-54).

Therefore, although it is known that MDM2/MDM4 heterodimer inhibits p53 function, heterodimer dissociation has not been assumed a current suitable strategy in order to favor p53 apoptotic activity. This due to the fact that MDM2 release, i.e. the main p53 inhibitor, could however carry out inhibiting function against p53. For this reason, current strategies are directed to the dissociation of p53 from MDM2 and MDM4 and not to hetorodimer dissociation.

The authors of the present invention have demonstrated that MDM2 opposes the pro-apoptotic activity of MDM4 and that, when MDM4/MDM2 dissociation or partial dissociation at specific binding sites occurs as result of stress conditions, the p53-mediated apoptotic pathway is promoted, independently of MDM2. These results are coherent with the model proposed by Shmueli indicating p53 regulators not only for control of p53 activity, but also as having an important role in p53-mediated choice between cell life and death (Shmueli A, Oren M. Mdm2: p53's lifesaver? Mol Cell. 2007 Mar 23;25(6):794-6;Mitochondrial MDM4 (MDMX): An unpredicted role in the p53-mediated intrinsic apoptotic pathway. Mancini F, Moretti F. Cell Cycle. 2009 Dec; 8(23):3854-9). In particular, the inventors of the present invention observed that the over-expression of MDM4 mutant (C437G), unable of binding MDM2, induces the activation of p53, and this activation of p53 is not inhibited by concomitant expression of MDM2. Moreover, the over-expression of MDM4 (exceeding MDM2 levels) in a model of transgenic mouse causes an increase of cellular death (apoptosis) as a result of gamma irradiation and delays the tumor appearance induced by mouse exposure to genotoxic agents.

On the basis of these data, MDM4/MDM2 interaction region has been characterized. Amino acids involved in MDM4/MDM2 binding have been identified and tested by molecular mutagenesis. L430, A434 in MDM2 and L433, 1489, F488 residues in MDM4, respectively, have been identified as relevant for MDM2/MDM4 complex.

Therefore, peptides belonging to the region of MDM4/MDM2 interaction and able to impair the inhibiting activity of MDM2/MDM4 heterodimer towards p53, that is to restore the p53 oncosuppressive function in cancer cells still harboring p53 wild type protein (approximately 50% of human tumors), and to maintain the proapoptotic function of MDM4 directing therefore the p53 response towards the apoptosis, have been prepared. Currently the majority of human tumors is characterized in that 50% express wild type and 50% express mutated p53, respectively. Therefore, independently from tumor type, this approach is directed to tumors expressing wild type p53.

In particular, three peptides of 12 amino acids (12 mer) have been prepared and tested at molecular and biological level. Since the peptides display some limitations for therapeutic application because of intrinsic instability thereof, chemical modifications necessary to increase the bioavailability and allow the in vivo administration have been carried out.

Techniques in order to increase the stability and bioavailability of peptides and allow administration thereof in vivo are well known to those skilled in the art. Particularly, above said peptides could be modified by means of insertion of hydrocarbon bridge suitable to increase the alpha helicity of said peptides (carbon staples). These modifications are able to promote cellular uptake, confer resistance to protease and increase global stability.

It is therefore a specific object of the present invention a peptide belonging to (i.e. comprised in, taking part of) MDM4, said peptide comprising the aminoacids isoleucine and phenylalanine of MDM4 position 489 and 488 and having the following general formula I:

R-KVFI-R1 (I)

wherein
R is chosen from the group consisting of I, VI, LVI, QLVI (SEQ ID NO:13), IQLVI (SEQ ID NO:14), EIQLVI (SEQ ID NO:15), KEIQLVI (SEQ ID NO:16), KKEIQLVI (SEQ ID NO:17), CKKEIQLVI (SEQ ID NO:18), ICKKEIQLVI (SEQ ID NO:19), PICKKEIQLVI (SEQ ID NO:20), CPICKKEIQLVI (SEQ ID NO:21) or hydrogen of amino functional group of K;
R1 is chosen from the group consisting of A or hydroxyl of carboxylic functional group of I;
wherein said R and R1 are chosen so that said peptide ranges from 5 to 16 amino acids, preferably, from 8 to 12. Preferably, the peptide belonging to the sequence of MDM4, at the levels of MDM4 and MDM2 interaction domain, can consist of the following sequence from amino acid 479 to amino acid 490 of MDM4:
KEIQLVIKVFIA (SEQ ID NO: 3).

An isolated nucleotide sequence encoding for peptide as above defined is a further object of the present invention.

Moreover, the invention concerns an expression vector comprising nucleotide sequence as above defined.

The invention refers also to a pharmaceutical composition comprising or consisting of at least one peptide, or at least one nucleotide sequence, or at least one vector as above defined, as an active ingredient, in association with one or more pharmaceutically acceptable adjuvant and/or excipients.

A peptide, nucleotide sequence, vector or pharmaceutical composition, as defined above, for use in the treatment of tumors preferably expressing wild type p53, or associated pathologies like hyperproliferative benign syndromes represent a further object of the present invention.

Said peptides according to the invention can, for example, be administered by intravenous way, or using liposomal vectors directly deliverable to the tumor, or, at last, by means of nano-particles. In addition, said peptides can be delivered also by gene therapy using DNA expression vectors.

As above reported, peptides of the invention can be modified according to known methods in order to increase stability and bioavailability of the peptides and allow in vivo administration thereof. Particularly, said peptides can be modified by insertion of hydrocarbon bridge suitable to increase alpha helicity thereof (carbon staples). As reported in experimental section, the tested peptides have been modified as below:
peptide 1: FITC-Ahx-ESSLPLNAIEPS-CONH2
peptide 2: FITC-Ahx-MEDSQNLLKPSS- CONH2
peptide 3: FITC-Ahx-KEIQLVIKVFIA-CONH2
wherein the Ahx term indicates a linker. In addition, the peptides have been modified, for example, also with acetyl in the place of FITC-Ahx:
peptide 1: Acet - ESSLPLNAIEPS-CONH2
peptide 2: Acet - MEDSQNLLKPSS- CONH2
peptide 3: Acet- KEIQLVIKVFIA-CONH2

The present invention now will be described by an illustrative, but not limitative way, according to preferred embodiments thereof, with detailed reference to enclosed drawings, wherein:

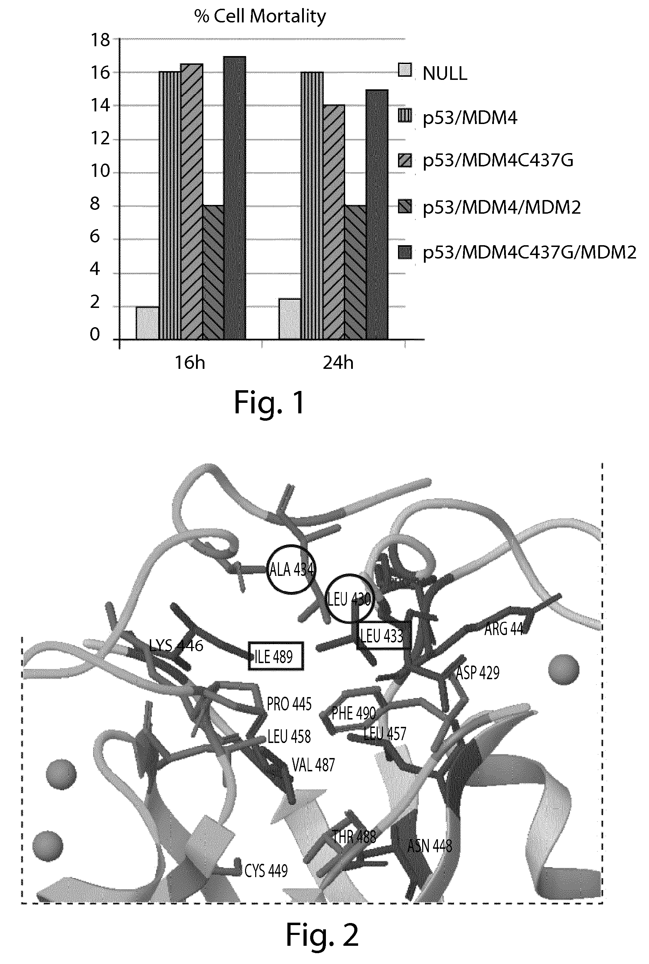

**Figure 1** shows cell death analysis as a result of over-expression of MDM4+p53, or MDM4C437G+p53 in the presence or absence of MDM2, in embryonic murine Mdm4^{-/-} p53^{-/-} (DNX) fibroblasts compared to control cells transfected using an empty vector (null). The percentage of dead cells has been calculated as ratio of Trypan blue positive cells to all counted cells. The average of 2 different experiments is reported.
**Figure 2** shows the binding interface of the two MDM4 and MDM2 RING finger domains. Boxed (in MDM4) and circled (in MDM2) positions represent potential key amino acids for this interaction.

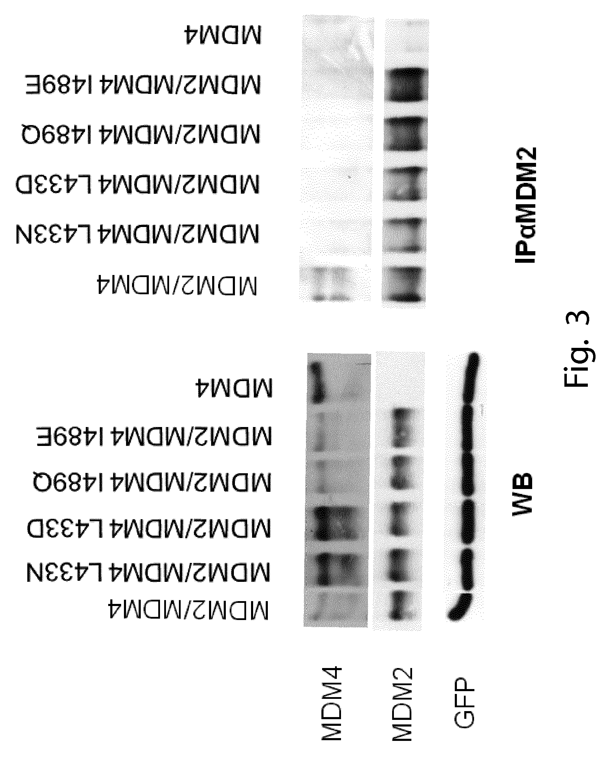

**Figure 3** shows the binding analysis of MDM2 to various MDM4 point mutants. DNX cells were transfected with expression vectors carrying MDM2-wt and MDM4-wt or its mutated forms (MDM4 L433N, L433D, I489Q, I489E) as shown in the upper part of the figure. Cell lysates have been collected and 700 µg of whole cell extracts have been immunoprecipitated with anti-MDM2 antibody and immunocomplexes analyzed by western blotting, as indicated in the figure (right panel). Left panel shows western blot analysis of a fraction of cellular lysates in order to control the expression levels of reported proteins.

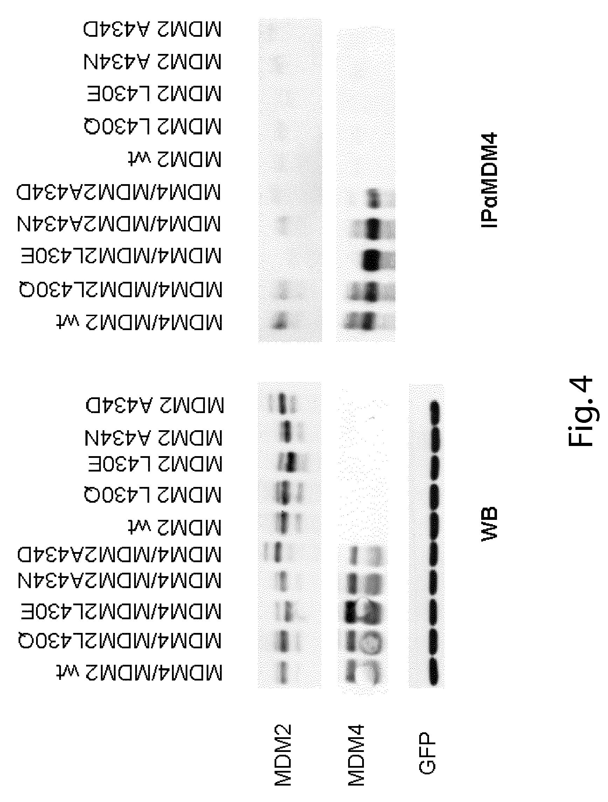

**Figure 4** shows the binding analysis of MDM4 to various MDM2point mutants. Murine embryonic Mdm2^{-/-}p53^{-/-}(DN1) fibroblasts were transfected with expression vectors carrying MDM4-wt and MDM2-wt or its mutated forms (MDM2-L430Q, L430E, A434N, A434D) as shown in the upper part of the figure. Cell lysates have been collected and 700 µg of whole cell extracts have been immunoprecipitated with anti-MDM4 antibody and immunocomplexes analyzed by western blotting, as indicated in the figure (right panel). Left panel shows western blot analysis of a fraction of cellular lysates in order to control the expression levels of reported proteins.

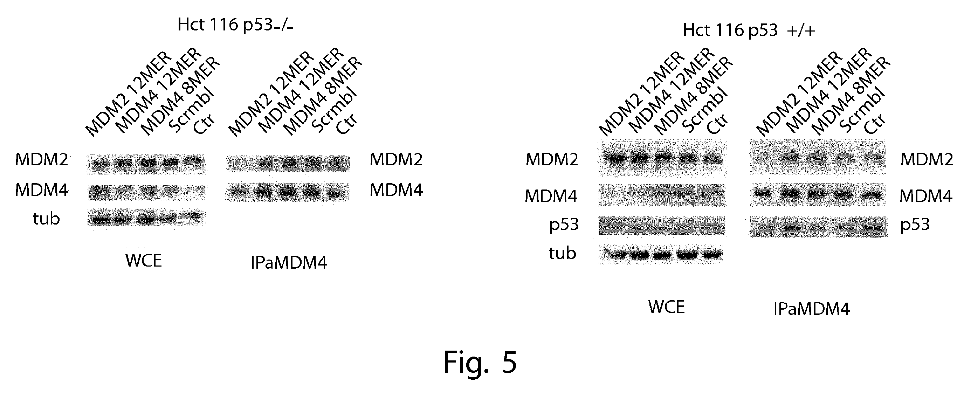

**Figure 5** shows the ability of peptide 1 to dissociate MDM4/MDM2 complex by immunoprecipitation assay. Transient transfection of peptides has been carried out in HCT116 wt and p53-/- colon carcinoma cell lines and the immunoprecipitation of MDM4 has been carried out on cell lysates in order to detect the presence of MDM2 within the immunocomplex, by western blot analysis.

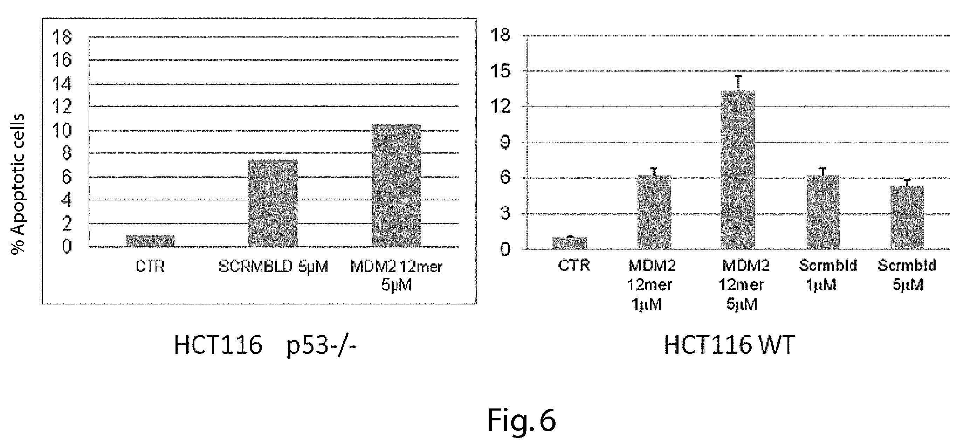

**Figure 6** shows the ability of peptide 1 to induce cell death. Cytofluorimetric analysis was carried out on HCT116 wt and p53-/- cells transfected with FITC tagged peptides and stained with Propidium Iodide (PI). Percentage of dead cells was evaluated specifically among the transfected cells (green population).

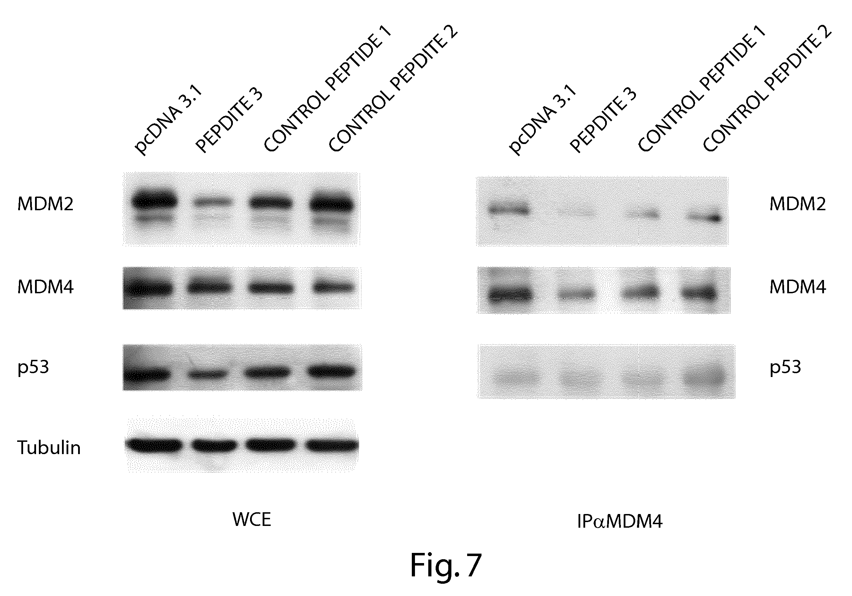

**Figure 7** shows the ability of peptide 3 to dissociate MDM4/MDM2 complex by immunoprecipitation assay. Transient transfection of peptides has been carried out in HCT116 wt and p53-/- colon carcinoma cell lines and the immunoprecipitation of MDM4 has been performed on cell lysates in order to detect the presence of MDM2 within the immunocomplex by western blot analysis.

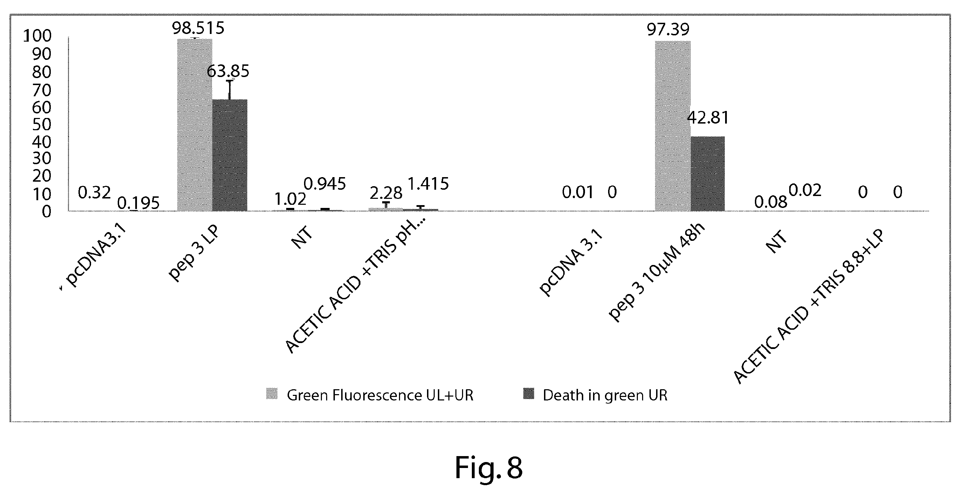

**Figure 8** shows the ability of peptide 3 to induce cell death. Cytofluorimetric analysis was performed on HCT116 wt and p53-/- cells transfected with FITC tagged peptides and stained with Propidium Iodide (PI). Percentage of dead cells was evaluated specifically among the transfected cells (green population).

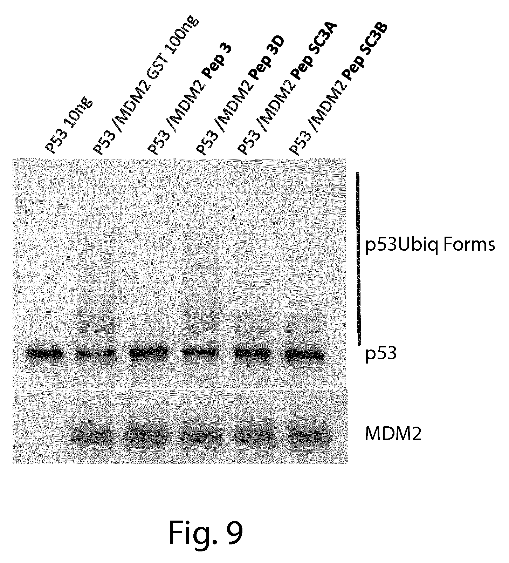

**Figure 9** shows in vitro ubiquitination assay of p53 performed by recombinant GST-MDM2. The figure shows the ability of Peptide 3, compared to control peptides (3D, SC3A and SC3B), to inhibit the ubiquitinatin activity of MDM2 towards p53.

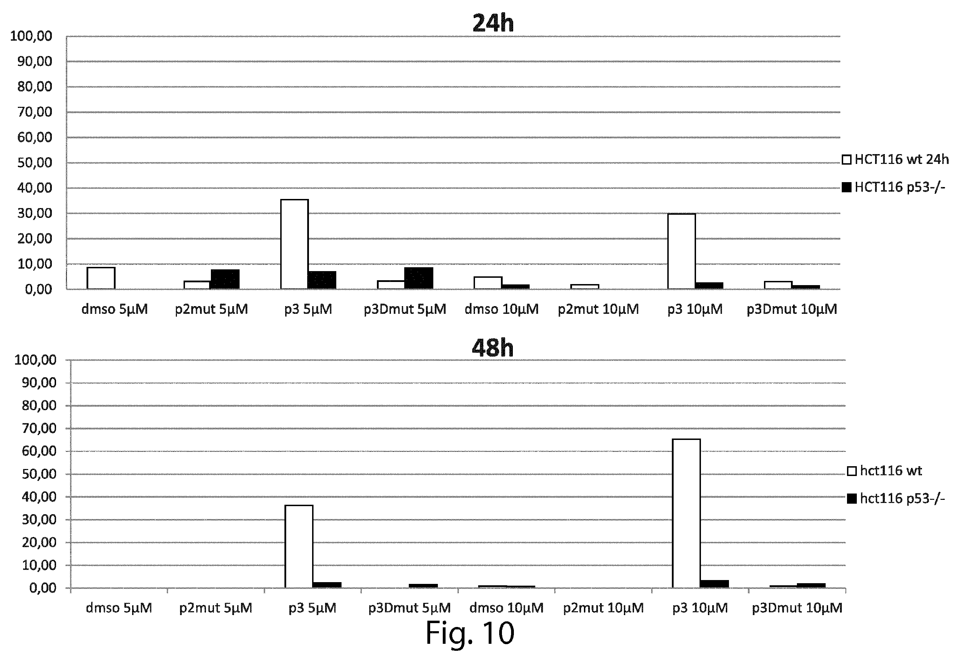

**Figure 10** shows the ability of peptide 3, compared to control peptides (3D and 2MUT), to induce apoptosis. In order to specifically detect apoptotic cells, TUNEL assay was carried out in HCT116 wt and p53-/- cells transfected with FITC tagged peptides. Percentage of apoptotic cells was evaluated specifically among the transfected cells (green population).

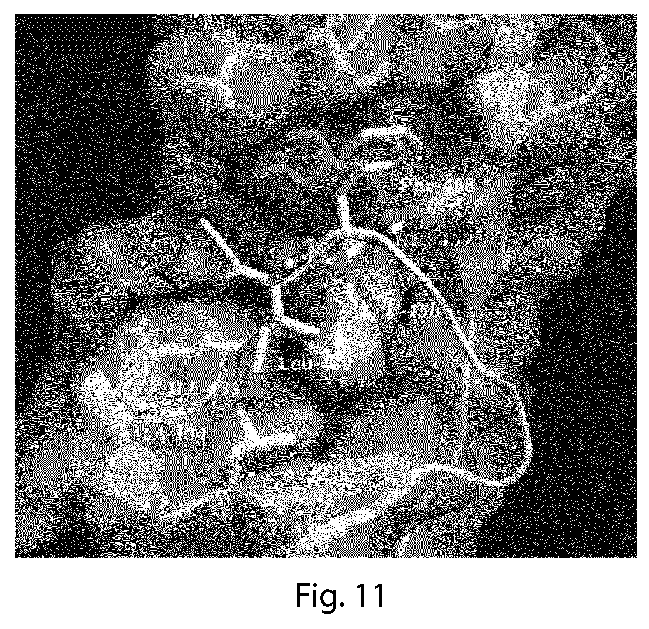

Figure 11 shows the binding mode of peptide 3 to MDM2 as resulting from molecular dynamic simulations. I489 occupies a hydrophobic cleft composed of A434, I435 and L458 of MDM2. F488 forms aromatic interactions with a H457 of MDM2 protein.

**Example 1:** *In vitro study about the effect of the dissociation of MDM4 from MDM2 on cellular death; preparation of peptides suitable to dissociate the heterodimer and in vitro study of apoptotic effectiveness thereof.*

### 1.1 Materials and methods

**Tranfections**. Transient transfections have been carried out using lipophilic vectors ("Lipofectamine 2000" for DNA, Invitrogen). This method is based on use of lipid vesicles (liposomes) mediating the transfer of DNA into eucaryotic cells.

Cells in 100 mm plates have been transfected with following vectors: pShutlle-hMDM4 (WT, C437G), pCMV-hMDM2 (WT), pCAG-p53.

Punctiform mutants have been obtained by PCR amplification with specific primers using plasmids containing hMdm4 and hMDM2 cDNAs and resulting products used for generation of plasmids containing hMdm4 and hMDM2 cDNAs using Qiagen mutagenesis kit.

### Immunoprecipitation.

Cells have been lysed using Saito lysis modified reagent (50 mM Tris-HCl, pH 7.4, 0.15 M NaCl, 0.5% Triton-X100, 5 mM EDTA). For p53 immunoprecipitation and BCL2 and MDM4 analysis EBC modified solution has been used (50 mM Tris-HCl, pH 7.4, 0.12 M NaCl, 0.4% NP-40, 1 mM EDTA, 1 Mm β-mercaptoethanol).

400µg of total protein extract has been incubated with protein G (Invitrogen) for 2 hours under stirring at 4°C in order not specific binding of proteins with said substrate can occur. Afterwards the cellular extract has been centrifuged, not specific complexes removed with precipitate and supernatant has been incubated for 16 hours with specific antibody. Successively, protein G has been added and incubated for 2 hour at 4°C. Finally, immunoprecipitate has been washed twice with Saito buffer and resuspended in 10µl of loading buffer solution (loading buffer 4X: 0,6 M β-mercaptoethanol; 8% SDS; 0,25 M Tris-HCl, pH 6,8; 40% glycerol; 0,2% Bromophenol blue). Samples have been denatured at 95°C for 5 minutes before loading on gel for electrophoresis and successive western blot analysis.

### Blot Western Analysis.

Different amounts (70-100 µg for samples to be analyzed) of total protein lysates have been separated by polyacrylamide gel electrophoresis (29% acrylamide and 1% bis-acrylamide) at 12% final concentration. Loading solution has been added to samples (loading buffer 4X: 0,6 M β-mercaptoethanol; 8% SDS; 0,25 M Tris-HCl, pH 6,8; 40% glycerol; 0,2% Bromophenol blue) suitable to denature definitively the same (as result of disruption of disulfide bridges by β-mercaptoethanol) and confer a definitive negative charge to all the proteins (by conjugation with SDS). Samples are boiled at 95°C for 5 minutes and successively loaded on gel. Electrophoretic run has been carried out at 100 V for approximately 2 hours in ionic buffer containing 25 mM Tris-HCl, pH 8,8, 200 mM glycine and 0.1 % SDS.

At the end of electrophoretic run proteins have been transferred on PVDF filter (Millipore) under electric field in buffer consisting of 25 mM Tris Base, 200 mM glycine and 20% methanol. Transfer of proteins from anode to cathode has been carried out for 3 hours at 70 V.

After proteins transfer, the filter has been incubated under stirring for 1 hour in TPBS buffer 1X (10 mM Tris-HCl, pH 7,6, 150 mM NaCl and 0,1% Tween 20) containing 5% skimmed milk powder (Bio-Rad) in order to block not specific antibody binding sites. The filter successively has been incubated for 2 hours with opportunely diluted primary antibody. At the end of the incubation the filter has been washed twice for approximately 10 minutes each with TPBS 1X and then incubated for 45 minutes with corresponding peroxidase-conjugated secondary antibody. After two washings for approximately 10 minutes with TPBS 1X, immunoreactivity has been evaluated using chemiluminescence reaction based on luminol oxidation (Amersham). Successively, chemiluminescence has been detected by autoradiography on sensitive plates (Kodak).

### 1.2 Effects of dissociation of MDM4 from MDM2

MDM2/MDM4 heterodimer is the main control tool for p53 levels and therefore inhibition thereof inside of cell under normal growth conditions (Wade and Wahl, 2009). Therefore it has been assumed that the dissociation of MDM4 from MDM2 can favor the implementation of pro-apoptotic function thereof, in addition to preventing degradation activity with respect to p53. The binding leading to heterodimer formation occurs at the level of "RING finger" domains of both the proteins (Tanimura et al., 1999). In order to point out the role of dissociation in the accomplishment of MDM4 pro-apoptotic activity, MDM4 mutant mutated at 437 cysteine residue has been used. Such mutation results in the impossibility to form a correct RING finger domain and thus MDM2 binding impossibility. Therefore, DNX cells have been transfected with constructs for expression of p53, MDM4 or MDM4C437G, and MDM2 proteins. Then Trypan Blue staining positive death cells have been counted and compared to number of total counted cells (Fig. 1). As it is apparent from obtained data MDM4 presence potentiates pro-apoptotic activity of p53, as previously observed and MDM4C437G expression acts analogously to MDM4-wt. However, MDM2 expression rescues cell death induced by co-expression of p53 and MDM4-wt, but not induced by MDM4C437G mutant. These data suggest, therefore, that factors suitable to prevent the association between MDM2 and MDM4 can avoid the functional inhibition of MDM2 and therefore potentiate the pro-apoptotic function of p53.

### 1.3 Preparation of peptides suitable to disrupt the interaction between MDM2 and MDM4

For design of peptide suitable to disrupt the interaction between MDM2 and MDM4, initially an analysis at crystallized structure interfaces of MDM2 and MDM4 RING domains (pdb code: 2vjf) has been carried out. This analysis, in particular, has been carried out using a software program (SiteMap, Schrodinger Software) suitable to characterize binding pockets in proteins. As result, two small binding pockets at level of MDM2 and MDM4 N-terminal regions, respectively, have been detected.

Human MDM4 and MDM2 protein sequences, MDM4 and MDM2 amino acid sequences of MDM4 and MDM2 interaction domains and MDM4 and MDM2 amino acid sequences occurring in crystallographic structure of MDM4/MDM2 dimer are below reported:
**MDM4 human protein sequence (SEQ ID NO: 4)**
**MDM4 sequence of MDM4-MDM2 interaction domain (SEQ ID NO: 5)**
**MDM4 sequence occurring in crystallographic structure of MDM4/MDM2 dimer (SEQ ID NO: 6)**
**MDM2 human protein sequence (SEQ ID NO: 7)**
**MDM2 sequence of MDM4-MDM2 interaction domain (SEQ ID NO: 8)**
**MDM2 sequence occurring in crystallographic structure of MDM4/MDM2 dimer (SEQ ID NO: 9)**

Starting from N-terminal regions of MDM2 and MDM4, the detection of key amino acid set involved in mediating interactions occurring at interface of MDM2/MDM4 complex has been carried out. Among these residues, Leu₄₃₀^{MDM2}, Ala₄₃₄^{MDM2}, Leu₄₃₃^{MDM4} and Ile₄₈₉^{MDM4} have been detected as particularly important for stabilization of heterodimeric complex of RING domain (Figure 2). In order to prove that said amino acids carry out a determining role in interaction of two proteins, the same have been replaced with other amino acid residues by point mutagenesis experiments. Mutations have been carried out in such a way to replace every amino acid of interest with an amino acid unable to form the association but, at the same time, maintaining unaltered the correct "folding" of the corresponding domain and thus of the protein. Moreover, in order to prove that the cause of possible dissociation was effectively the replaced and not inserted amino acid, every amino acid residue has been replaced with two different residues with different total charge. Thus amino acids with net negative side chain charge, i.e. glutamate and aspartate, and corresponding polar side chain amino acids, i.e. glutamine and asparagine, have been selected. Accordingly, eight different mutants by in vitro mutagenesis have been produced:
1. MDM4L433N
2. MDM4L433D
3. MDM4I489Q
4. MDM4I489E
5. MDM2L430Q
6. MDM2L430E
7. MDM2A434N
8. MDM2A434D

At this point, the ability of each mutant to interact with corresponding "wild-type" protein has been analyzed. DNX cells have been transfected with constructs expressing MDM2-wt protein and each of the four MDM4 mutants or MDM4-wt protein like control. Cells successively have been collected and resulting lysates immunoprecipitated with anti-MDM2 antibody and the immunocomplexes analyzed for the presence of MDM4 different forms (Fig. 3). Results have demonstrated that all and the four MDM4 mutants proved to be unable to bind MDM2, meanwhile it is very clear the binding of two "wild-type" proteins. This results indicates therefore that for MDM4 leucine in position 433 and isoleucine in position 489 are determinant for binding with MDM2 and therefore formation of heterodimer. In specular way, MDM2 mutants have been tested. Murine fibroplast cells derived from p53 and Mdm2 (DN1) "knockout" mouse have been transfected with constructs expressing MDM4 protein and each of different mutants of MDM2 or MDM2-wt like control. After 24 hours, cells have been collected and corresponding lysates immunoprecipitated using anti-MDM4 antibody (Fig. 4). According to western blot analysis of this immunoprecipitation it can be observed, as expected, that MDM2-wt binds MDM4 meanwhile MDM2 L430E and A434D mutants are unable to bind MDM4 under the same conditions. MDM2 L430Q and A434N mutants instead prove to be able to bind MDM4 although in lower extent than MDM2-wt protein. This result indicates therefore that 430 leucine and 434 alanine are two determining sites for MDM2 and MDM4 binding. However the insertion in these sites of an amino acid with weakly polar side chain clearly does not compromise remarkably the MDM2 ability to bind MDM4 while the insertion of amino acids with net negative side chain charge is suitable to dissociate the complex completely, suggesting that at level of these sites the bind is localized on side chain of involved amino acids. Starting from these residues, three MDM2 and MDM4 12mer peptides, corresponding to following amino acid sequences (SEQ ID NO: 1, SEQ ID NO: 2 and SEQ ID NO: 3), have been designed:
1) ESSLPLNAIEPC i.e. Glu₁-Ser₂-Ser₃-Leu₄-Pro₅-Leu₆-Asn₇-Ala₈-Ile₉-Glu₁₀-Pro₁₁-Cys₁₂ (SEQ ID NO:1)
2) MEDCQNLLKPCS i.e. Met₁-Glu₂-Asp₃-Cys₄-Gln₅-Asn₆-Leu₇-Leu₈-Lys₉-Prol₁₀-Cys₁₁-Ser₁₂ (SEQ ID NO:2)
3) KEIQLVIKVFIA i.e. Lys₁-Glu₂-Ile₃-Gln₄-Leu₅-Val₆-Ile₇-Lys₈-Val₉-Phe₁₀-Ile₁₁-Ala₁₂ (SEQ ID NO:3)

Since the alpha helix conformation stability (helicity) is important parameter in order to favor a strong interaction between peptides and MDM2 and MDM4 RING domains, helicity of peptides 1 and 2 using simulations of molecular dynamics has been evaluated. These simulations have been carried out using Desmond software program and OPLS 2005 force field, as implemented according to Schrodinger software package. Particularly, simulations have carried out for 20 ns time length in aqueous solvent, at a temperature of 300°K, and according to NPT Berendesen protocol. Trajectories obtained by two simulations then have been analyzed through the calculation of secondary structure of peptides during said 20 ns. Resulting peptide helicities, expressed as percentage, has been 23,7% and 12,5% for peptide 1 and peptide 2, respectively. It has not been possible a similar analysis to be carried out for peptide 3.

### 1.4 Study on apoptotic effectiveness of peptide 1 and peptide 3

SEQ ID NO: 1-3 peptides have been modified by addition of FITC at N-terminal residue followed by a spacer. Amide group, in order to increase peptide stability, has been attached at C-terminal residue:
Peptide 1: FITC-Ahx-ESSLPLNAIEPS-CONH2
Peptide 2: FITC-Ahx-MEDSQNLLKPSS- CONH2
Peptide 3: FITC-Ahx-KEIQLVIKVFIA-CONH2
   Ahx term indicates a linker.
   Below properties thereof are shown.

**Peptide 1 (SEQ ID NO: 1):** the peptide has been tested by immunoprecipitation assays and detection of cell death.

It has been investigated whether peptides were able to enter the cell simply by addition thereof at given concentration (5µM) to cell culture medium and analyzing for the presence thereof by confocal and fluorescence microscopy, each peptide being labeled with green fluorophore (FAM). It has been observed that peptides were detected in cells only after transfection by liposomes. Successively, the ability of peptides to disrupt the binding by MDM4 or MDM2 immunoprecipitation after peptide transfection in HCT116 cancer cell line, and detection of presence of corresponding partner in immunocomplex by western blot assay have been evaluated. Two HCT116 wild type and HCT116 p53 -/syngeneic cell lines have been used in order to understand also observed effect dependence on the presence of p53. SEQ ID NO:1 peptide proved to be suitable to dissociate said two proteins and induce a significant apoptotic response (Fig. 5, 6). Of interest, this response was significantly higher in comparison to the control Scramble peptide in HCT116 p53+/+ cells but not in HCT116 p53-/- cells, indicating the specific activity of this peptide in the presence of p53 (Fig. 6). Accordingly, this peptide was able to preserve the binding of p53 to MDM4 (Fig. 5).

**Peptide 3 (SEQ ID NO: 3):** analogously to peptide 1, also for this peptide the ability to penetrate the cell has been evaluated by counting the number of cells positive for the presence of FITC fluorophore. This peptide has been proved to be internalized in cells both by liposomal vectors and independently although with lower efficiency. Successively, ability thereof to dissociate MDM4/MDM2 complex has been analyzed by immunoprecipitation experiments. We have observed that the peptide is highly effective in dissociating said two molecules while does not dissociate p53 from MDM4 (Figure 7).

Moreover, this peptide has been proved to be highly effective in inducing cellular apoptosis in specific way since another peptide unable to dissociate MDM4/MDM2 does not induce cell death (not shown data). Apoptotic effect in addition is dependent on p53 since induced death is meaningfully lower in p53 lacking than harboring cells (Figure 8).

### Example 2: Study about the role of peptide 3 (SEQ ID NO:3) composition in its molecular and biological activity

Further experiments have been performed to define the role of peptide 3 composition in its molecular and biological activity. These experiments have demonstrated that aminoacids Isoleucine at position 489 and Phenylalanine at position 488 of MDM4 protein are essential for the ability of Peptide 3 to inhibit MDM4/MDM2 activity and apoptosis-promoting function. Indeed, a peptide with two substitutions at position 10 and 11 of peptide 3 (Peptide 3D, KEIQLVIKVAEA (SEQ ID NO:38) was completely ineffective in inhibiting ubiquitination of p53 and inducing apoptosis (Figure 9 and 10). Similarly, two peptides composed with the same aminoacids of peptide 3 but arranged in different order (Peptide SC3A, VQEAFKLIKIVI (SEQ ID NO:39) and Peptide SC3B, AIKIFVKVLEIQ (SEQ ID NO:40) are ineffective in inhibiting ubiquitination of p53 (Figure 9), confirming that the aminoacid Isoleucine at position 11 and Phenylalanine at position 10 are crucial contact points for inhibition of MDM4/MDM2 heterodimer activity.

Supporting these new experimental data about the importance of Isoleucine 489 and Phenylalanine 488, molecular dynamic simulations have shown that these residues engage in specific hydrophobic and aromatic interactions with MDM2 (Figure 11). In particular, Isoleucine 489 is harbored in a hydrophobic cleft composed of Alanine 434, Isoleucine 435 and Leucine 458 of MDM2 protein. Likewise, Phenylalanine 488 forms aromatic interactions with a Histidine residue (HID-457) of MDM2 protein.

In addition, since SC3A and SC3B have different solubility and helical content (as estimated by AGADIR software), these data reinforce the importance of targeting aa A488 and I489, independently of other peptide properties.

## Claims

1. Peptide belonging to MDM4, said peptide comprising the aminoacids isoleucine and phenylalanine of MDM4 position 489 and 488 and having the following general formula I:
R-KVFI-R1 (I)
wherein
R is chosen from the group consisting of I, VI, LVI, QLVI (SEQ ID NO:13), IQLVI (SEQ ID NO:14), EIQLVI (SEQ ID NO:15), KEIQLVI (SEQ ID NO:16), KKEIQLVI (SEQ ID NO:17), CKKEIQLVI (SEQ ID NO:18), ICKKEIQLVI (SEQ ID NO:19), PICKKEIQLVI (SEQ ID NO:20), CPICKKEIQLVI (SEQ ID NO:21) or hydrogen of amino functional group of K;
R1 is chosen from the group consisting of A or hydroxyl of carboxylic functional group of I;
wherein said R and R1 are chosen so that said peptide ranges from 5 to 16 amino acids, preferably, from 8 to 12.

2. Peptide according to claim 1, wherein said peptide consists of the following MDM4 sequence from amino acid 479 to amino acid 490:
KEIQLVIKVFIA (SEQ ID NO:3).

3. Isolated nucleotide sequence codifying a peptide as defined in anyone of claims from 1 to 2.

4. Expression vector comprising the nucleotide sequence as defined in claim 3.

5. Pharmaceutical composition comprising or consisting of at least one peptide as defined in anyone of the claims from 1 to 2, or at least one nucleotide sequence as defined in claim 3, or at least one vector as defined in claim 4, as active ingredient, in association with one or more pharmaceutically acceptable adjuvant and/or excipients.

6. Peptide as defined in anyone of the claims from 1 to 2, nucleotide sequence as defined in claim 3, vector as defined in claim 4, or pharmaceutical composition as defined in claim 5, for use in the treatment of tumors.

7. Peptide as defined in anyone of the claims from 1 to 2, nucleotide sequence as defined in claim 3, vector as defined in claim 4, or pharmaceutical composition as defined in claim 5, for use according to claim 6, wherein the tumors are wild type p53 expressing tumors.

8. Peptide as defined in anyone of the claims from 1 to 2, nucleotide sequence as defined in claim 3, vector as defined in claim 4, or pharmaceutical composition as defined in claim 5, for use in the treatment of hyperproliferative benign syndromes.

## Patentansprüche

1. Peptid, welches zu MDM4 gehört, wobei das Peptid die Aminosäuren Isoleucin und Phenylalanin an der MDM4-Position 489 und 488 umfasst und die folgende allgemeine Formel I aufweist:
R-KVFI-R1 (I)
wobei R ausgewählt ist aus der Gruppe, die aus I, VI, LVI, QLVI (SEQ-ID-Nr. 13), IQLVI (SEQ-ID-NR: 14), EIQLVI(SEQ-ID-NR. 15), KEIQLVI (SEQ-ID-NR. 16); KKEIQLVI (SEQ-ID-NR. 17), CKKEIQLVI (SEQ-ID-NR. 18), ICKKEIQLVI (SEQ-ID-NR. 19), PICKKEIQLVI (SEQ-ID-NR. 20), CPICKKEIQLVI(SEQ-ID-NR. 21) oder Wasserstoff der aminofunktionellen Gruppe von K besteht;
R1 ausgewählt ist aus der Gruppe, welche aus A oder dem Hydroxyl der carboxyfunktionellen Gruppe von I besteht;
wobei R und R1 so ausgewählt sind, dass die Peptide im Bereich von 5 bis 16 Aminosäuren, vorzugsweise von 8 bis 12 Aminosäuren, liegen.

2. Peptid nach Anspruch 1, wobei das Peptid aus der folgenden MDM4-Sequenz von Aminosäure 479 bis Aminosäure 490 besteht:
KEIQLVIKVFIA (SEQ-ID-NR. 3).

3. Isolierte Nucleotidsequenz, die für ein Peptid, wie in irgendeinem der Ansprüche 1 bis 2 definiert, kodiert.

4. Expressionsvektor, welcher die Nukleotidsequenz, wie in Anspruch 3 definiert, umfasst.

5. Pharmazeutische Zusammensetzung, umfassend oder bestehend aus mindestens einem Peptid wie in irgendeinem der Ansprüche 1 bis 2 definiert, oder mindestens eine Nukleotidsequenz wie in Anspruch 3 definiert, oder mindestens ein Vektor, wie in Anspruch 4 definiert, als aktiven Bestandteil in Verbindung mit einem oder mehreren pharmazeutisch annehmbaren Adjuvans/Adjuvantien und/oder Excipient/Excipienten.

6. Peptid wie in irgendeinem der Ansprüche 1 bis 2 definiert, Nukleotidsequenz wie in Anspruch 3 definiert, Vektor wie in Anspruch 4 definiert oder pharmazeutische Zusammensetzung wie in Anspruch 5 definiert, zur Verwendung bei der Behandlung von Tumoren.

7. Peptid wie in irgendeinem der Ansprüche 1 bis 2 definiert, Nukleotidsequenz wie in Anspruch 3 definiert, Vektor wie in Anspruch 4 definiert oder pharmazeutische Zusammensetzung wie in Anspruch 5 definiert, zur Verwendung nach Anspruch 6, wobei die Tumore Wildtyp p53-exprimierende Tumore sind.

8. Peptid wie in irgendeinem der Ansprüche 1 bis 2 definiert, Nukleotidsequenz wie in Anspruch 3 definiert, Vektor wie in Anspruch 4 definiert, oder pharmazeutische Zusammensetzung wie in Anspruch 5 definiert, zur Verwendung bei der Behandlung von gutartigen Hyperproliferationssyndromen.

## Revendications

1. Peptide appartenant à MDM4, ledit peptide comprenant les acides aminés isoleucine et phénylalanine de position MDM4 489 et 488 et ayant la formule générale suivante I :
R-KVFI-R1 (I)
dans laquelle
R est choisi à partir du groupe constitué par I, VI, LVI, QLVI (SEQ ID n° 13), IQLVI (SEQ ID n° 14), EIQLVI (SEQ ID n° 15), KEIQLVI (SEQ ID n° 16), KKEIQLVI (SEQ ID n° 17), CKKEIQLVI (SEQ ID n° 18), ICKKEIQLVI (SEQ ID n° 19), PICKKEIQLVI (SEQ ID n° 20), CPICKKEIQLVI (SEQ ID n° 21) ou un hydrogène de groupe fonctionnel amino de K;
R1 est choisi à partir du groupe constitué par A ou un hydroxyle de groupe fonctionnel carboxylique de I ;
dans lequel lesdits R et R1 sont choisis de sorte que ledit peptide s'étale sur 5 à 16 acides aminés, de préférence, 8 à 12.

2. Peptide selon la revendication 1, dans lequel ledit peptide consiste en la séquence MDM4 suivante allant de l'acide aminé 479 à l'acide aminé 490 :
KEIQLVIKVFIA (SEQ ID n° 3).

3. Séquence de nucléotide isolé codifiant un peptide selon l'une quelconque des revendications 1 à 2.

4. Vecteur d'expression comprenant la séquence de nucléotide selon la revendication 3.

5. Composition pharmaceutique comprenant ou consistant en au moins un peptide selon l'une quelconque des revendications 1 à 2, ou au moins une séquence de nucléotide selon la revendication 3, ou au moins un vecteur selon la revendication 4, en tant qu'ingrédient actif, en association avec un ou plusieurs adjuvants et/ou excipients acceptables d'un point de vue pharmaceutique.

6. Peptide selon l'une quelconque des revendications 1 à 2, séquence de nucléotide selon la revendication 3, vecteur selon la revendication 4, ou composition pharmaceutique selon la revendication 5, pour une utilisation dans le traitement de tumeurs.

7. Peptide selon l'une quelconque des revendications 1 à 2, séquence de nucléotide selon la revendication 3, vecteur selon la revendication 4, ou composition pharmaceutique selon la revendication 5, pour une utilisation selon la revendication 6, dans lequel les tumeurs sont des tumeurs exprimant le type sauvage p53.

8. Peptide selon l'une quelconque des revendications 1 à 2, séquence de nucléotide selon la revendication 3, vecteur selon la revendication 4, ou composition pharmaceutique selon la revendication 5, pour une utilisation dans le traitement de syndromes bénins hyperproliférants.


### SEQUENCE LISTING

<110> Consiglio Nazionale delle Ricerche Università degli Studi di Perugia
<120> Peptides able to impair the inhibiting activity of MDM2/MDM4 heterodimer towards p53 and use thereof for cancer treatment
<130> BE29013
<150> RM2012A000060
   <151> 2012-02-21
<160> 40
<170> PatentIn version 3.5
<210> 1
   <211> 12
   <212> PRT
   <213> Artificial Sequence
<220>
   <223> MDM2 sequence of the interaction domain between MDM4 and MDM2 from aa 427 to aa 438 of MDM2
<400> 1
   Glu Ser Ser Leu Pro Leu Asn Ala Ile Glu Pro Cys
   1               5                   10
<210> 2
   <211> 12
   <212> PRT
   <213> Artificial Sequence
<220>
   <223> MDM4 sequence of the interaction domain between MDM4 and MDM2 from aa 427 to aa 438 of MDM4
<400> 2
   Met Glu Asp Cys Gln Asn Leu Leu Lys Pro Cys Ser
   1               5                   10
<210> 3
   <211> 12
   <212> PRT
   <213> Artificial Sequence
<220>
   <223> MDM4 sequence of the interaction domain between MDM4 and MDM2 from aa 479 to aa 490 of MDM4
<400> 3
   Lys Glu Ile Gln Leu Val Ile Lys Val Phe Ile Ala
   1               5                   10
<210> 4
   <211> 490
   <212> PRT
   <213> Artificial Sequence
<220>
   <223> Human MDM4 protein sequence
<400> 4
   Met Thr Ser Phe Ser Thr Ser Ala Gln Cys Ser Thr Ser Asp Ser Ala
   1               5                   10                  15
   Cys Arg Ile Ser Pro Gly Gln Ile Asn Gln Val Arg Pro Lys Leu Pro
               20                  25                  30
   Leu Leu Lys Ile Leu His Ala Ala Gly Ala Gln Gly Glu Met Phe Thr
           35                  40                  45
   Val Lys Glu Val Met His Tyr Leu Gly Gln Tyr Ile Met Val Lys Gln
       50                  55                  60
   Leu Tyr Asp Gln Gln Glu Gln His Met Val Tyr Cys Gly Gly Asp Leu
   65                  70                  75                  80
   Leu Gly Glu Leu Leu Gly Arg Gln Ser Phe Ser Val Lys Asp Pro Ser
                   85                  90                  95
   Pro Leu Tyr Asp Met Leu Arg Lys Asn Leu Val Thr Leu Ala Thr Ala
               100                 105                 110
   Thr Thr Asp Ala Ala Gln Thr Leu Ala Leu Ala Gln Asp His Ser Met
           115                 120                 125
   Asp Ile Pro Ser Gln Asp Gln Leu Lys Gln Ser Ala Glu Glu Ser Ser
       130                 135                 140
   Thr Ser Arg Lys Arg Thr Thr Glu Asp Asp Ile Pro Thr Leu Pro Thr
   145                 150                 155                 160
   Ser Glu His Lys Cys Ile His Ser Arg Glu Asp Glu Asp Leu Ile Glu
                   165                 170                 175
   Asn Leu Ala Gln Asp Glu Thr Ser Arg Leu Asp Leu Gly Phe Glu Glu
               180                 185                 190
   Trp Asp Val Ala Gly Leu Pro Trp Trp Phe Leu Gly Asn Leu Arg Ser
           195                 200                 205
   Asn Tyr Thr Pro Arg Ser Asn Gly Ser Thr Asp Leu Gln Thr Asn Gln
       210                 215                 220
   Asp Val Gly Thr Ala Ile Val Ser Asp Thr Thr Asp Asp Leu Trp Phe
   225                 230                 235                 240
   Leu Asn Glu Ser Val Ser Glu Gln Leu Gly Val Gly Ile Lys Val Glu
                   245                 250                 255
   Ala Ala Asp Thr Glu Gln Thr Ser Glu Glu Val Gly Lys Val Ser Asp
               260                 265                 270
   Lys Lys Val Ile Glu Val Gly Lys Asn Asp Asp Leu Glu Asp Ser Lys
           275                 280                 285
   Ser Leu Ser Asp Asp Thr Asp Val Glu Val Thr Ser Glu Asp Glu Trp
       290                 295                 300
   Gln Cys Thr Glu Cys Lys Lys Phe Asn Ser Pro Ser Lys Arg Tyr Cys
   305                 310                 315                 320
   Phe Arg Cys Trp Ala Leu Arg Lys Asp Trp Tyr Ser Asp Cys Ser Lys
                   325                 330                 335
   Leu Thr His Ser Leu Ser Thr Ser Asp Ile Thr Ala Ile Pro Glu Lys
               340                 345                 350
   Glu Asn Glu Gly Asn Asp Val Pro Asp Cys Arg Arg Thr Ile Ser Ala
           355                 360                 365
   Pro Val Val Arg Pro Lys Asp Ala Tyr Ile Lys Lys Glu Asn Ser Lys
       370                 375                 380
   Leu Phe Asp Pro Cys Asn Ser Val Glu Phe Leu Asp Leu Ala His Ser
   385                 390                 395                 400
   Ser Glu Ser Gln Glu Thr Ile Ser Ser Met Gly Glu Gln Leu Asp Asn
                   405                 410                 415
   Leu Ser Glu Gln Arg Thr Asp Thr Glu Asn Met Glu Asp Cys Gln Asn
               420                 425                 430
   Leu Leu Lys Pro Cys Ser Leu Cys Glu Lys Arg Pro Arg Asp Gly Asn
           435                 440                 445
   Ile Ile His Gly Arg Thr Gly His Leu Val Thr Cys Phe His Cys Ala
       450                 455                 460
   Arg Arg Leu Lys Lys Ala Gly Ala Ser Cys Pro Ile Cys Lys Lys Glu
   465 470 475 480
   Ile Gln Leu Val Ile Lys Val Phe Ile Ala
   485 490
<210> 5
   <211> 78
   <212> PRT
   <213> Artificial Sequence
<220>
   <223> MDM4 sequence of the interaction domain between MDM4 and MDM2 from aa 413 to aa 490 of MDM4
<400> 5
   Gln Leu Asp Asn Leu Ser Glu Gln Arg Thr Asp Thr Glu Asn Met Glu
   1 5 10 15
   Asp Cys Gln Asn Leu Leu Lys Pro Cys Ser Leu Cys Glu Lys Arg Pro
   20 25 30
   Arg Asp Gly Asn Ile Ile His Gly Arg Thr Gly His Leu Val Thr Cys
   35 40 45
   Phe His Cys Ala Arg Arg Leu Lys Lys Ala Gly Ala Ser Cys Pro Ile
   50 55 60
   Cys Lys Lys Glu Ile Gln Leu Val Ile Lys Val Phe Ile Ala
   65 70 75
<210> 6
   <211> 63
   <212> PRT
   <213> Artificial Sequence
<220>
   <223> MDM4 sequence of the interaction domain between MDM4 and MDM2 from aa 428 to aa 490 of MDM4
<400> 6
   Glu Asp Cys Gln Asn Leu Leu Lys Pro Cys Ser Leu Cys Glu Lys Arg
   1 5 10 15
   Pro Arg Asp Gly Asn Ile Ile His Gly Arg Thr Gly His Leu Val Thr
   20 25 30
   Cys Phe His Cys Ala Arg Arg Leu Lys Lys Ala Gly Ala Ser Cys Pro
   35 40 45
   Ile Cys Lys Lys Glu Ile Gln Leu Val Ile Lys Val Phe Ile Ala
   50 55 60
<210> 7
   <211> 491
   <212> PRT
   <213> Artificial Sequence
<220>
   <223> Human MDM2 protein sequence
<400> 7
   Met Cys Asn Thr Asn Met Ser Val Pro Thr Asp Gly Ala Val Thr Thr
   1               5                   10                  15
   Ser Gln Ile Pro Ala Ser Glu Gln Glu Thr Leu Val Arg Pro Lys Pro
               20                  25                  30
   Leu Leu Leu Lys Leu Leu Lys Ser Val Gly Ala Gln Lys Asp Thr Tyr
           35                  40                  45
   Thr Met Lys Glu Val Leu Phe Tyr Leu Gly Gln Tyr Ile Met Thr Lys
       50                  55                  60
   Arg Leu Tyr Asp Glu Lys Gln Gln His Ile Val Tyr Cys Ser Asn Asp
   65                  70                  75                  80
   Leu Leu Gly Asp Leu Phe Gly Val Pro Ser Phe Ser Val Lys Glu His
                   85                  90                  95
   Arg Lys Ile Tyr Thr Met Ile Tyr Arg Asn Leu Val Val Val Asn Gln
               100                 105                 110
   Gln Glu Ser Ser Asp Ser Gly Thr Ser Val Ser Glu Asn Arg Cys His
           115                 120                 125
   Leu Glu Gly Gly Ser Asp Gln Lys Asp Leu Val Gln Glu Leu Gln Glu
       130                 135                 140
   Glu Lys Pro Ser Ser Ser His Leu Val Ser Arg Pro Ser Thr Ser Ser
   145                 150                 155                 160
   Arg Arg Arg Ala Ile Ser Glu Thr Glu Glu Asn Ser Asp Glu Leu Ser
                   165                 170                 175
   Gly Glu Arg Gln Arg Lys Arg His Lys Ser Asp Ser Ile Ser Leu Ser
               180                 185                 190
   Phe Asp Glu Ser Leu Ala Leu Cys Val Ile Arg Glu Ile Cys Cys Glu
           195                 200                 205
   Arg Ser Ser Ser Ser Glu Ser Thr Gly Thr Pro Ser Asn Pro Asp Leu
   210 215 220
   Asp Ala Gly Val Ser Glu His Ser Gly Asp Trp Leu Asp Gln Asp Ser
   225 230 235 240
   Val Ser Asp Gln Phe Ser Val Glu Phe Glu Val Glu Ser Leu Asp Ser
   245 250 255
   Glu Asp Tyr Ser Leu Ser Glu Glu Gly Gln Glu Leu Ser Asp Glu Asp
   260 265 270
   Asp Glu Val Tyr Gln Val Thr Val Tyr Gln Ala Gly Glu Ser Asp Thr
   275 280 285
   Asp Ser Phe Glu Glu Asp Pro Glu Ile Ser Leu Ala Asp Tyr Trp Lys
   290 295 300
   Cys Thr Ser Cys Asn Glu Met Asn Pro Pro Leu Pro Ser His Cys Asn
   305 310 315 320
   Arg Cys Trp Ala Leu Arg Glu Asn Trp Leu Pro Glu Asp Lys Gly Lys
   325 330 335
   Asp Lys Gly Glu Ile Ser Glu Lys Ala Lys Leu Glu Asn Ser Thr Gln
   340 345 350
   Ala Glu Glu Gly Phe Asp Val Pro Asp Cys Lys Lys Thr Ile Val Asn
   355 360 365
   Asp Ser Arg Glu Ser Cys Val Glu Glu Asn Asp Asp Lys Ile Thr Gln
   370 375 380
   Ala Ser Gln Ser Gln Glu Ser Glu Asp Tyr Ser Gln Pro Ser Thr Ser
   385 390 395 400
   Ser Ser Ile Ile Tyr Ser Ser Gln Glu Asp Val Lys Glu Phe Glu Arg
   405 410 415
   Glu Glu Thr Gln Asp Lys Glu Glu Ser Val Glu Ser Ser Leu Pro Leu
   420 425 430
   Asn Ala Ile Glu Pro Cys Val Ile Cys Gln Gly Arg Pro Lys Asn Gly
   435 440 445
   Cys Ile Val His Gly Lys Thr Gly His Leu Met Ala Cys Phe Thr Cys
   450 455 460
   Ala Lys Lys Leu Lys Lys Arg Asn Lys Pro Cys Pro Val Cys Arg Gln
   465                 470                 475                 480
   Pro Ile Gln Met Ile Val Leu Thr Tyr Phe Pro
                   485                 490
<210> 8
   <211> 81
   <212> PRT
   <213> Artificial Sequence
<220>
   <223> MDM2 sequence of the interaction domain between MDM4 and MDM2 from aa 411 to aa 491 of MDM2
<400> 8
   Val Lys Glu Phe Glu Arg Glu Glu Thr Gln Asp Lys Glu Glu Ser Val
   1               5                   10                  15
   Glu Ser Ser Leu Pro Leu Asn Ala Ile Glu Pro Cys Val Ile Cys Gln
               20                  25                  30
   Gly Arg Pro Lys Asn Gly Cys Ile Val His Gly Lys Thr Gly His Leu
           35                  40                  45
   Met Ala Cys Phe Thr Cys Ala Lys Lys Leu Lys Lys Arg Asn Lys Pro
       50                  55                  60
   Cys Pro Val Cys Arg Gln Pro Ile Gln Met Ile Val Leu Thr Tyr Phe
   65                  70                  75                  80
   Pro
<210> 9
   <211> 64
   <212> PRT
   <213> Artificial Sequence
<220>
   <223> MDM2 sequence of the interaction domain between MDM4 and MDM2 from aa 428 to aa 491 of MDM2
<400> 9
   Ser Ser Leu Pro Leu Asn Ala Ile Glu Pro Cys Val Ile Cys Gln Gly
   1               5                   10                  15
   Arg Pro Lys Asn Gly Cys Ile Val His Gly Lys Thr Gly His Leu Met
               20                  25                  30
   Ala Cys Phe Thr Cys Ala Lys Lys Leu Lys Lys Arg Asn Lys Pro Cys
   35 40 45
   Pro Val Cys Arg Gln Pro Ile Gln Met Ile Val Leu Thr Tyr Phe Pro
   50 55 60
<210> 10
   <211> 18
   <212> PRT
   <213> Artificial Sequence
<220>
   <223> MDM4 sequence of the interaction domain between MDM4 and MDM2 from aa 473 to aa 490 of MDM4
<400> 10
   Ser Cys Pro Ile Cys Lys Lys Glu Ile Gln Leu Val Ile Lys Val Phe
   1 5 10 15
   Ile Ala
<210> 11
   <211> 31
   <212> PRT
   <213> Artificial Sequence
<220>
   <223> MDM4 sequence of the interaction domain between MDM4 and MDM2 from aa 418 to aa 448 of MDM4
<400> 11
   Ser Glu Gln Arg Thr Asp Thr Glu Asn Met Glu Asp Cys Gln Asn Leu
   1 5 10 15
   Leu Lys Pro Cys Ser Leu Cys Glu Lys Arg Pro Arg Asp Gly Asn
   20 25 30
<210> 12
   <211> 35
   <212> PRT
   <213> Artificial Sequence
<220>
   <223> MDM2 sequence of the interaction domain between MDM4 and MDM2 from aa 415 to aa 449 of MDM2
<400> 12
   Glu Arg Glu Glu Thr Gln Asp Lys Glu Glu Ser Val Glu Ser Ser Leu
   1 5 10 15
   Pro Leu Asn Ala Ile Glu Pro Cys Val Ile Cys Gln Gly Arg Pro Lys
   20 25 30
   Asn Gly Cys
   35
<210> 13
   <211> 4
   <212> PRT
   <213> Artificial Sequence
<220>
   <223> MDM4 sequence of the interaction domain between MDM4 and MDM2 from aa 82 to aa 85 of MDM4
<400> 13
   Gln Leu Val Ile
   1
<210> 14
   <211> 5
   <212> PRT
   <213> Artificial Sequence
<220>
   <223> MDM4 sequence of the interaction domain between MDM4 and MDM2 from aa 81 to aa 85 of MDM4
<400> 14
   Ile Gln Leu Val Ile
   1 5
<210> 15
   <211> 6
   <212> PRT
   <213> Artificial Sequence
<220>
   <223> MDM4 sequence of the interaction domain between MDM4 and MDM2 from aa 80 to aa 85 of MDM4
<400> 15
   Glu Ile Gln Leu Val Ile
   1 5
<210> 16
   <211> 7
   <212> PRT
   <213> Artificial Sequence
<220>
   <223> MDM4 sequence of the interaction domain between MDM4 and MDM2 from aa 79 to aa 85 of MDM4
<400> 16
   Lys Glu Ile Gln Leu Val Ile
   1 5
<210> 17
   <211> 8
   <212> PRT
   <213> Artificial Sequence
<220>
   <223> MDM4 sequence of the interaction domain between MDM4 and MDM2 from aa 78 to aa 85 of MDM4
<400> 17
   Lys Lys Glu Ile Gln Leu Val Ile
   1               5
<210> 18
   <211> 9
   <212> PRT
   <213> Artificial Sequence
<220>
   <223> MDM4 sequence of the interaction domain between MDM4 and MDM2 from aa 77 to aa 85 of MDM4
<400> 18
   Cys Lys Lys Glu Ile Gln Leu Val Ile
   1               5
<210> 19
   <211> 10
   <212> PRT
   <213> Artificial Sequence
<220>
   <223> MDM4 sequence of the interaction domain between MDM4 and MDM2 from aa 76 to aa 85 of MDM4
<400> 19
   Ile Cys Lys Lys Glu Ile Gln Leu Val Ile
   1               5                   10
<210> 20
   <211> 11
   <212> PRT
   <213> Artificial Sequence
<220>
   <223> MDM4 sequence of the interaction domain between MDM4 and MDM2 from aa 75 to aa 85 of MDM4
<400> 20
   Pro Ile Cys Lys Lys Glu Ile Gln Leu Val Ile
   1               5                   10
<210> 21
   <211> 12
   <212> PRT
   <213> Artificial Sequence
<220>
   <223> MDM4 sequence of the interaction domain between MDM4 and MDM2 from aa 74 to aa 85 of MDM4
<400> 21
   Cys Pro Ile Cys Lys Lys Glu Ile Gln Leu Val Ile
   1               5                   10
<210> 22
   <211> 4
   <212> PRT
   <213> Artificial Sequence
<220>
   <223> MDM2 sequence of the interaction domain between MDM4 and MDM2 from aa 26 to aa 29 of MDM2
<400> 22
   Val Glu Ser Ser
   1
<210> 23
   <211> 5
   <212> PRT
   <213> Artificial Sequence
<220>
   <223> MDM2 sequence of the interaction domain between MDM4 and MDM2 from aa 25 to aa 29 of MDM2
<400> 23
   Ser Val Glu Ser Ser
   1               5
<210> 24
   <211> 6
   <212> PRT
   <213> Artificial Sequence
<220>
   <223> MDM2 sequence of the interaction domain between MDM4 and MDM2 from aa 24 to aa 29 of MDM2
<400> 24
   Glu Ser Val Glu Ser Ser
   1               5
<210> 25
   <211> 7
   <212> PRT
   <213> Artificial Sequence
<220>
   <223> MDM2 sequence of the interaction domain between MDM4 and MDM2 from aa 23 to aa 29 of MDM2
<400> 25
   Glu Glu Ser Val Glu Ser Ser
   1               5
<210> 26
   <211> 8
   <212> PRT
   <213> Artificial Sequence
<220>
   <223> MDM2 sequence of the interaction domain between MDM4 and MDM2 from aa 22 to aa 29 of MDM2
<400> 26
   Lys Glu Glu Ser Val Glu Ser Ser
   1 5
<210> 27
   <211> 9
   <212> PRT
   <213> Artificial Sequence
<220>
   <223> MDM2 sequence of the interaction domain between MDM4 and MDM2 from aa 21 to aa 29 of MDM2
<400> 27
   Asp Lys Glu Glu Ser Val Glu Ser Ser
   1 5
<210> 28
   <211> 10
   <212> PRT
   <213> Artificial Sequence
<220>
   <223> MDM2 sequence of the interaction domain between MDM4 and MDM2 from aa 20 to aa 29 of MDM2
<400> 28
   Gln Asp Lys Glu Glu Ser Val Glu Ser Ser
   1 5 10
<210> 29
   <211> 11
   <212> PRT
   <213> Artificial Sequence
<220>
   <223> MDM2 sequence of the interaction domain between MDM4 and MDM2 from aa 19 to aa 29 of MDM2
<400> 29
   Thr Gln Asp Lys Glu Glu Ser Val Glu Ser Ser
   1 5 10
<210> 30
   <211> 4
   <212> PRT
   <213> Artificial Sequence
<220>
   <223> MDM2 sequence of the interaction domain between MDM4 and MDM2 from aa 35 to aa 38 of MDM2
<400> 30
   Ile Glu Pro Cys
   1
<210> 31
   <211> 5
   <212> PRT
   <213> Artificial Sequence
<220>
   <223> MDM2 sequence of the interaction domain between MDM4 and MDM2 from aa 35 to aa 39 of MDM2
<400> 31
   Ile Glu Pro Cys Val
   1               5
<210> 32
   <211> 6
   <212> PRT
   <213> Artificial Sequence
<220>
   <223> MDM2 sequence of the interaction domain between MDM4 and MDM2 from aa 35 to aa 40 of MDM2
<400> 32
   Ile Glu Pro Cys Val Ile
   1               5
<210> 33
   <211> 7
   <212> PRT
   <213> Artificial Sequence
<220>
   <223> MDM2 sequence of the interaction domain between MDM4 and MDM2 from aa 35 to aa 41 of MDM2
<400> 33
   Ile Glu Pro Cys Val Ile Cys
   1               5
<210> 34
   <211> 8
   <212> PRT
   <213> Artificial Sequence
<220>
   <223> MDM2 sequence of the interaction domain between MDM4 and MDM2 from aa 35 to aa 42 of MDM2
<400> 34
   Ile Glu Pro Cys Val Ile Cys Gln
   1               5
<210> 35
   <211> 9
   <212> PRT
   <213> Artificial Sequence
<220>
   <223> MDM2 sequence of the interaction domain between MDM4 and MDM2 from aa 35 to aa 43 of MDM2
<400> 35
   Ile Glu Pro Cys Val Ile Cys Gln Gly
   1               5
<210> 36
   <211> 10
   <212> PRT
   <213> Artificial Sequence
<220>
   <223> MDM2 sequence of the interaction domain between MDM4 and MDM2 from aa 35 to aa 44 of MDM2
<400> 36
   Ile Glu Pro Cys Val Ile Cys Gln Gly Arg
   1               5                   10
<210> 37
   <211> 11
   <212> PRT
   <213> Artificial Sequence
<220>
   <223> MDM2 sequence of the interaction domain between MDM4 and MDM2 from aa 35 to aa 45 of MDM2
<400> 37
   Ile Glu Pro Cys Val Ile Cys Gln Gly Arg Pro
   1               5                   10
<210> 38
   <211> 12
   <212> PRT
   <213> Artificial Sequence
<220>
   <223> MDM4 sequence of the interaction domain between MDM4 and MDM2 from aa 479 to aa 490 of MDM4 in which FI in positions 10 and 11 of said sequence has been substituted with AE
<400> 38
   Lys Glu Ile Gln Leu Val Ile Lys Val Ala Glu Ala
   1               5                   10
<210> 39
   <211> 12
   <212> PRT
   <213> Artificial Sequence
<220>
   <223> peptide composed with the same aminoacids of SEQ ID NO:3 but arranged in different order
<400> 39
   Val Gln Glu Ala Phe Lys Leu Ile Lys Ile Val Ile
   1               5                   10
<210> 40
   <211> 12
   <212> PRT
   <213> Artificial Sequence
<220>
   <223> peptide composed with the same aminoacids of SEQ ID NO:3 but arranged in different order
<400> 40
   Ala Ile Lys Ile Phe Val Lys Val Leu Glu Ile Gln
   1               5                   10